# EUROPEAN PATENT APPLICATION

(11) **EP 2 309 260 A1**
(43) Date of publication of application: **13.04.2011**
(21) Application number: 10177182.2
(22) Date of filing: 16.09.2010
(51) Int. Cl.: G01N 33/50

(54) **Method for screening of active tuberculosis**

(30) Priority: 18.09.2009 US 243824 P; 30.08.2010 US 871161
(71) Applicant: Becton, Dickinson and Company, Franklin Lakes, NJ 07417 (US)
(72) Inventor: Park, Eun Ju, Los Altos, CA 94022 (US); Nomura, Laurel, San Jose, CA 95123 (US)
(74) Representative: von Kreisler Selting Werner

(57) **Abstract**

The present invention provides an improved and simplified assay for use in diagnosing active Tuberculosis infections. The present assay is carried out using a sample of whole blood, does not require separating the blood into components, such as the isolation of peripheral blood mononucleocytes (PBMC), and is carried out using only cell-surface staining of T-cells.

## Description

### Cross Reference To Related Applications

This application claims priority to U.S. provisional application No. 61/243,824, filed September 18, 2009, which is incorporated herein by reference.

### BACKGROUND OF THE INVENTION.

### FIELD OF THE INVENTION

The present invention relates to the fields of immunology and medical microbacteriology. More particularly, the present invention relates to the detection and evaluation of an active mycobacterial infection, more particularly active infection with *Mycobacterium tuberculosis.*

### DESCRIPTION OF RELATED ART

The diagnosis of acute tuberculosis is usually based on characteristic X-ray findings, clinical symptoms, and positive sputum-smear or culture. However, even if the sputum-smear is negative, patients suspected to have active pulmonary TB will frequently be hospitalized and treated until culture results are obtained. This is an unnecessary burden on the patient and public spending if cultures turn out negative.

T-cell responses to the relatively M. tuberculosis-specific ESAT-6 protein are used in the detection of latent tuberculosis infection, but have been reported not to be reliable for the diagnosis of acute TB. T-cell responses to tuberculin have no diagnostic value for detecting actute TB in BCG vaccinees unless very strongly positive.

Streitz et al., 2007, Loss of Receptor on Tuberculin-Reactive T-Cells Marks Active Pulmonary Tuberculosis, PLoS ONE 2(8): e735, incorporated herein by reference, describe a immunological test for active pulmonary tuberculosis. They demonstrated that CD27 expression on CD4⁺ T-cells activated in response to stimulation with tuberculin can be used to detect active pulmonary TB. In the described methods, peripheral blood mononuclear cells, also referred to as peripheral blood mononucleocytes (PBMC) were stimulated ex-vivo for 16 hours with tuberculin (purified protein derivate, or PPD), and CD27 expression among the subset of CD4⁺ T-cells that responded to stimulation was measured by flow cytometry. In patients with active clinical pulmonary TB, the population of tuberculin-specific CD4⁺ T-cells was observed to exhibit a larger proportion of CD27⁻ cells than in BCG-vaccinated individuals, individuals with a latent TB infection, or in unexposed individuals. CD4⁺ T-cells that responded to stimulation with PPD were identified by the expression of both cell-surface markers and an intracellular marker, which required the simultaneous staining of both cell-surface and intracellular markers.

### BRIEF SUMMARY OF THE INVENTION.

The present invention provides an improved and simplified assay for use in diagnosing active tuberculosis infections. The present assay is carried out using a sample of whole blood without the need of separating the blood into components, such as the isolation of peripheral blood mononuclear cells (PBMC). The assay is a further simplified relative to previously described assays in that it requires only cell-surface staining of T-cells.

The methods of the present invention of detecting an active mycobaterial infection in a patient involve contacting a sample of whole blood from the patient with Mycobacterium tuberculosis-specific antigen, at least one labeled antibody that identifies CD4⁺ T-cells, at least one labeled antibody that identifies activated T-cells, and a labeled antibody that is specific for CD27. The sample is then analyzed to detect the relative numbers of CD27⁻ T-cells and CD27⁺ T-cells within the activated CD4⁺ T-cell subset.

Preferred Mycobacterium tuberculosis-specific antigens include tuberculin, purified protein derivate (PPD) tuberculin, ESAT-6, CFP-10, and TB 7.7.

In preferred embodiments of the invention, activated T-cells are identified by the expression of CD40L. Other cell-surface activation markers, such as CD69, are known in the art and may be used in the present methods.

The assay of the present invention can distinguish active vs. latent TB infections, it can be used in both pulmonary a non-pulminary infections, and is suitable for use both with adult and with pediatric patients.

The resurgence of TB infections in the world today results in part to the prevalence of AIDS. The flow cytometric assay of the present invention also is well suited for use in conjunction with a CD4⁺ T-cell assays used as a measure of disease state of HIV-infected patients.

### BRIEF DESCRIPTION OF THE SEVERAL VIEWS OF THE DRAWINGS

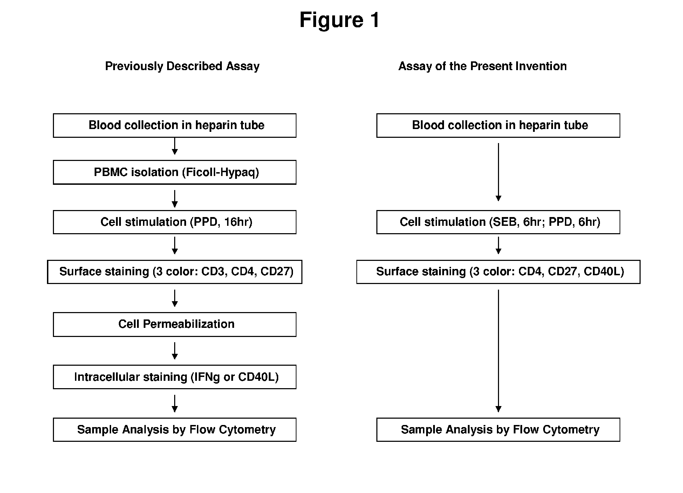

Figure 1 provides flow charts comparing the major steps of the prior art method of detecting active TB infection with an embodiment of the improved methods of the present invention. Details of these methods are further described in the text.
Figure 2 provides a comparison of results obtained using the prior art method of detecting active TB infection with an embodiment of the improved methods of the present invention.
Figure 3 provides a comparison of results obtained using the prior art method of detecting active TB infection with an embodiment of the improved methods of the present invention.
Figure 4 provides assay results showing the classification of CD4⁺ T-cells based on the expression levels of CD27 and CD40L, using the methods of the present invention in which both markers are measured using cell-surface staining only.

### DETAILED DESCRIPTION OF THE INVENTION.

In order that the invention herein described may be fully understood, a number of terms are explicitly defined, below. Terms not explicitly defined are intended to have their usual meaning in the fields of cell biology and cytometry. Flow cytometry is described at length in the extensive literature in this field, including, for example, Landy et al. (eds.), Clinical Flow Cytometry, Annals of the New York Academy of Sciences Volume 677 (1993); Bauer et al. (eds), Clinical Flow Cytometry: Principles and Applications, Williams & Wilkins (1993); Ormerod (ed.), Flow Cytometry: A Practical Approach, Oxford Univ. Press (1997);

Jaroszeski et al. (eds.), Flow Cytometry Protocols, Methods in Molecular Biology No. 91, Humana Press (1997); and Practical Shapiro, Flow Cytometry, 4th ed., Wiley-Liss (2003); all incorporated herein by reference.

By "whole blood" is intended a fluid blood sample as drawn in the presence of an anticoagulant from a mammal and substantially unfractionated thereafter.

"Antibody" includes all products, derived or derivable from antibodies or from antibody genes, that are useful as markers in the flow cytometric methods described herein. "Antibody" thus includes, inter alia, natural antibodies, antibody fragments, antibody derivatives, and genetically-engineered antibodies, antibody fragments, and antibody derivatives.

"Cell subset-defining antibody" refers to any antibody that may be used, alone or in combination with other antibodies, to facilitate identification of a particular subset of cells, and thus includes antibodies that are specific for epitopes displayed by cells of the subset. Typically, cell subsets may be identified by the presence of particular markers expressed by the cell and/or the absence of particular markers. By "absence" is intended a level of expression, as measured in an immunoassay, such as a flow cytometric assay, that is not significantly different from background.

"T-cell subset-defining antibody" refers to cell subset-defining antibody for the identification of a particular subset of T-cells (also known as T-lymphocytes). In the present methods, antibodies specific for cell-surface molecules are used to identify CD4⁺ T-cells. To identify CD4+ T-cells, typically multiple antibodies are used, including a CD4-specific antibody and at least one antibody that identifies T-cells, such as a CD3-specific antibody. Alternatively, CD4+ T-cells can be identified as CD8⁻, CD3⁺ cells. Other T-cell identifying antibodies, or combinations of antibodies, are well known in the art. In particular, the extensive literature on CD4 tests for monitoring AIDS patients describes as variety of antibody combinations that identify T-cells, and which may be used in the present methods.

A "cell activator", as used herein, refers to any substance that is capable of inducing or upregulating protein expression of a cell, including expression of cytokines, chemokines, cell surface proteins, or viral proteins from endogenous viral sequences. Suitable cell activators for use in the present invention are described below.

In the present methods, one or more Mycobacterium tuberculosis-specific antigens is used as a T-cell activator. Preferably, tuberculin, purified protein derivate (PPD) tuberculin, early secretory protein (ESAT)-6, culture filtrate protein (CFP)-10, or TB 7.7 is used. As used herein, Mycobacterium tuberculosis-specific antigen is meant to encompass the protein or proteins and fragments thereof, whether derived from the organism or from an expression vector using standard recombinant molecular biology techniques. Fusion proteins of multiple Mtb-specific antigens also can be used.

Antibodies are delineated by the standard CD nomenclature for the target molecule (see, e.g., Knapp W, Dorken K, Gilks WR, et al., eds. Leukocyte typing IV: white cell differentiation antigens. Oxford: Oxford University Press, 1989). For convenience, CD designations (e.g., CD4) are used herein to refer to not only the antibody cluster of differentiation, but also the corresponding antigen. This usage is common in the scientific literature, and one of skill in the art will understand the usage from the context.

### Kits

The reagents of the present invention can be advantageously used as a kit for carrying out the method of the invention and could be employed in a variety of applications, e.g., as kits suitable for scientific, medical and/or diagnostic purposes. The manufacture of the kits follows preferably standard procedures that are known to the person skilled in the art. Kits can advantageously include instructions for use.

### EXAMPLES

In the description that follows, it is to be understood that the various particular combinations of reagents described below are considered to be embodiments of the invention, and that the invention is not limited to the particular combinations exemplified.

The present invention is additionally described by way of the following illustrative, nonlimiting examples, that provide a better understanding of the present invention and of its many advantages.

### Example 1

### Sample Preparation Protocol

This example describes a preferred sample preparation protocol for carrying out the methods of the present invention using whole blood samples.

### I. Whole blood sample

Draw blood into a heparinized Vacutainer™ tube, and hold at room temperature until use (no more than ∼4 hrs).

### II. Activation

The protocol described below is designed for duplicates of each test condition; amounts of sample may be adjusted as long as reagent proportions remain identical. Use of more than 1 ml of blood per tube is not recommended.
1. For each patient sample, prepare one appropriately labeled round-bottom polypropylene tube (e.g. 15 ml Falcon #352096, or 14 ml Falcon #352059; BD Biosciences, San Jose, CA) for each sample treatment: (1) an unstimulated negative control, (2) a PPD-stimulated test sample, and (3) an SEB (Streptococcal enterotoxin B)-stimulated positive control.
2. Add 500 µl of whole blood into each tube.
3. Add 500 ng of CD40-specific mAb into each tube. A preferred CD40-specific mAb is produced from clone G28.5 (available, for example, from Dendritics, Lyon, France). The CD40-specific mAb acts as a blocker to facilitate subsequent staining of CD40L in step III, below.
4.
   a) To unstimulated sample(s), add nothing.
   b) To create a PPD-stimulated sample, add 5 µg of Tuberculin PPD (e.g., RT50, Statens Serum Institut, Copenhagen, Denmark).
   c) To create an SEB-stimulated sample, add 1.25 µg SEB (e.g. #S4881, Sigma, St. Louis, MO).
5. Vortex samples, cap loosely, and transfer to a 37°C, 5% CO₂ incubator. For best results (but not essential), hold tubes at 5-degree slant, especially when using more than 500 µl blood per tube. Incubate for six hours (increasing the incubation time up to about 16 hours may be preferable). Preferably, staining of the sample should be carried out immediately following activation. However, the samples may be held at 4°C for up to 12 hours. Alternately, samples may be incubated in a programmable water bath at 37°C for six hours, then held in the water bath at 4° - 18°C for up to 12 hours. Typically, a water bath holds the tubes upright.

### III. Staining

As described herein, the preferred staining reagents include the following labeled antibodies: CD3-V450 (alternatively, CD3-FITC), CD4-PerCP-Cy5.5, CD27-PE, and CD40L-APC. The particular dye combinations used are not a critical aspect of the invention, and other dye combinations may be used.
1. Label two (for duplicate analysis) 12 x 75 mm polystyrene tubes (e.g. Falcon #352052) for each treated sample.
2. Vortex the samples vigorously for at least 30 seconds.
3. Add 150 µl of sample blood into each labeled 12 x 75 tube.
4. Apply staining mAbs to each tube: 250 ng CD3-V450, 60 ng CD4-PerCP-Cy5.5, 30 ng CD27-PE, and 60 ng CD40L-APC, all of which are available from BD Biosciences (San Jose, CA).
5. Incubate at room temperature, light-protected, for 30 minutes.
6. Add 1.5 ml BD FACS Lysing Solution (BD Biosciences, San Jose, CA) to each tube, vortex gently. Incubate at room temperature, light-protected, for 10 minutes.
7. Centrifuge tubes at 500 x g for five minutes. Decant supernatant by tipping tubes upside down, but not shaking.
8. Vortex tubes, add 2 ml BD FACS Wash Buffer (PBS + 0.5% BSA + 0.1% NaN₃), and centrifuge tubes at 500 x g for five minutes. Decant supernatant by tipping tubes upside down, but not shaking.
9. Repeat step 8. Vortex tubes following decantation.
10. Store tubes at 4°C, light-protected, until analyzed. Analysis should be carried out within 24 hours.

Alternatively, the addition of CD40-specific mAb in Step II(5) may be omitted, in which case the dye-labeled CD40L-specific mAb is added at this step, prior to activation. Preliminary testing indicates that this alternative method may give useable, but somewhat inferior results, but extensive optimization has not been carried out.

### Example 2

### Analysis by Flow Cytometry

The samples prepared as described in example 1 are analyzed using a flow cytometer. For use with the preferred choice of dyes, described above, a flow cytometer having three excitation lasers, a blue, a red, and a violet, is used. Examples of usable flow cytometers include the BD FACSCanto™ Flow Cytometer, the BD™ LSR II System, and the BD FACSAria™ II Cell Sorter, all available from BD Biosciences (San Jose, CA).

The flow cytometer is set up according to the manufacturer's instructions. The prepared samples are analyzed, and the cell populations of interest are identified and counted by appropriate gating using well-known methods.

CD4⁺ T-cells are identified by the expression of CD3 and CD4. Other combinations of markers that identify CD4⁺ T-cells, which are well known in the art, may be used to identify this cell subpopulation.

Activation of CD4⁺ T-cells is identified by the cell-surface expression of CD40L (also known as CD154) on the identified CD4⁺ T-cells (i.e., simultaneous expression of both the CD4⁺ T-cell identifying markers and the activation marker). Other cell-surface markers of CD4⁺ T-cell activation, which are well known in the art, may be used to identify this activated cell subpopulation.

The assay is used to diagnose an active TB infection based on the level of CD27 expression in the subset of CD4⁺ T-cells activated in response to stimulation with tuberculin (PPD), as described in Streitz et al., 2007, supra.

### Example 3

### Comparison of the present method with the prior art method.

The ability of the present methods to detect CD27 positive and negative populations among activated CD4⁺ T-cells was compared with the prior art methods. Positive-control samples from two healthy donors were analyzed using each method.

The present method was carried out essentially as described above using whole blood activated with SEB for 6 hours.

The prior art methods were essentially as described in Streitz et al., 2007, supra.

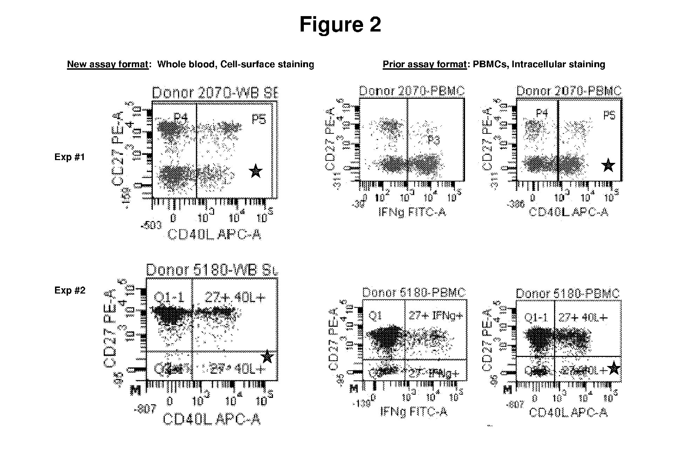

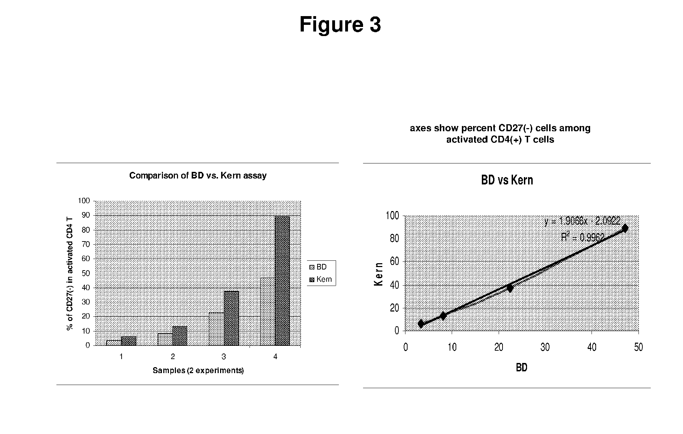

The data are shown in figures 2 and 3. In figure 2, the CD27⁻ populations are indicated with a star.

The data demonstrate that the methods of the present invention, which work with whole blood and with only cell-surface staining, provide results that are comparable to the results obtained using the prior art methods.

### Example 4

### Use of the present method

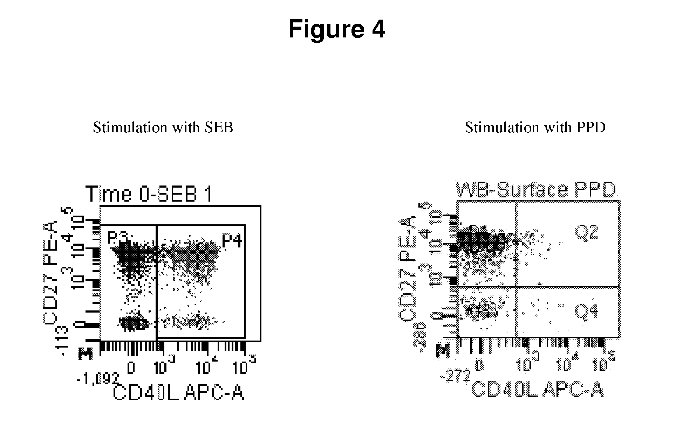

Figure 4 presents data from carrying out the methods of the present invention using both SEB-activated (positive control) samples and PPD-activated samples. The data show that the assay of the present invention enables a determination of the level of CD27 expression in a subset of CD4⁺ T-cells activated in response to stimulation with tuberculin (PPD).

## Claims

1. A method of detecting an active mycobaterial infection in a patient, comprising:
contacting a sample of whole blood from said patient with Mycobacterium tuberculosis-specific antigen, at least one labeled antibody that identifies CD4+ T-cells, at least one labeled antibody that identifies activated T-cells, and a labeled antibody that is specific for CD27; and
cytometrically detecting the relative numbers of CD27⁻ T-cells and CD27⁺ T-cells with the activated CD4⁺ T-cell subset.

2. The method of claim 1, wherein said Mycobacterium tuberculosis-specific antigen is selected from the group consisting of tuberculin, purified protein derivate (PPD) tuberculin, early secretory protein (ESAT)-6, culture filtrate protein (CFP)-10, and TB 7.7, and fragments thereof.

3. The method of claim 2, wherein said Mycobacterium tuberculosis-specific antigen is tuberculin or purified protein derivate (PPD) tuberculin.

4. The method of claim 1, wherein said at least one labeled antibody that identifies T-cells comprises a CD4-specific antibody.

5. The method of claim 1, wherein said at least one labeled antibody that identifies T-cells comprises a CD4-specific antibody and a CD3-specific antibody.

6. The method of claim 1, wherein said at least one labeled antibody that identifies activated cells comprises a CD40L-specific antibody.
